# EUROPEAN PATENT APPLICATION

(11) **EP 4 497 459 A1**
(43) Date of publication of application: **29.01.2025**
(21) Application number: 23773431.4
(22) Date of filing: 05.01.2023
(51) Int. Cl.: A61M 5/32, A61M 5/178

(54) **NEEDLE-SHIELDED PREFILLED SYRINGE**

(30) Priority: 21.03.2022 CN 202210276597
(71) Applicant: Shanghai Innopac Medical Technology Co., Ltd., Shanghai 201605 (CN)
(72) Inventor: GUO, Rong, Shanghai 201605 (CN)
(74) Representative: IPrime Bonnekamp Sparing Patentanwaltsgesellschaft mbH
(86) International application number: PCT/CN2023/070631
(87) International publication number: WO 2023/179179

(57) **Abstract**

The present disclosure belongs to the technical field of prefilled syringes. Provided is a needle-shielded prefilled syringe. By means of a syringe barrel, a needle, a needle mounting member, a shield unit and a needle protector unit, the technical effect of encasing the needle so as to prevent a syringe from stabbing other people during disposal is achieved. The present disclosure has the following advantages: firstly, the shield unit is utilized to encase the needle, so that the needle is not exposed to stab other people, and while the needle is exposed, the shield unit does not detach from the barrel; secondly, a plurality of stopping hooks are provided, so that the effect that the shield unit is fixed on a circumferential surface of the barrel when being opened is achieved, and the shield unit does not slide down during an injection process.

## Description

### TECHNICAL FIELD

The present disclosure belongs to the technical field of prefilled syringes, and particularly relates to a needle-shielded prefilled syringe.

### BACKGROUND ART

A prefilled syringe is a novel medicine packaging form, and may be understood as "an injectable medicine package" from another perspective. Since the prefilled syringe is also a type of syringe, the risk of stabbing people when a syringe needle is discarded after use also exists. Therefore, in order to solve the problem, a device for destroying a needle before the needle is discarded is available on the market. For example, Chinese Utility Model Patent (Patent No. CN210936412U, published on July 7, 2020 has disclosed a needle shearing device of an integrated syringe. The device comprises a first shearing member, wherein a first cavity extending in an axial direction of the first shearing member is defined in the first shearing member, one end of the first cavity is open, the first cavity can accommodate a syringe needle and allow the syringe to penetrate through, a first shearing opening communicated with the first cavity is formed in the other end of the first shearing member, and the needle and a connecting portion can penetrate through the first shearing opening; a first blade, wherein the first blade is arranged at the first shearing opening; and a second shearing member, wherein a second cavity extending in an axial direction of the second shearing member is defined in the second shearing member, one end of the second cavity is open, the other end of the second cavity sleeves the other end of the first shearing member, a driving portion is arranged in the second shearing member, the second shearing member can rotate around an axis thereof to drive the connecting portion by the driving portion to abut against the first blade and be broken off by the first blade.

However, such shearing device has the following defects: firstly, injection work in hospitals is huge, and even a nurse needs to visit each ward to complete an injection task, it is very inappropriate to require the nurse to carry the shearing device, and the nurse cannot ensure that he/she will remember to carry the shearing device at any time; secondly, the needle may fly out in the shearing process, resulting in loss of the needle and even direct injuries to other people; and thirdly, the sheared needle still needs to be discarded, although the volume of medical wastes can be greatly reduced and centralized treatment can be done, the problem of needle exposure when the needle is discarded is not fundamentally solved, and centralized treatment is also time-consuming and laborious.

Therefore, in conclusion, in order to solve the problem, a prefilled syringe that ensures that a needle thereof cannot be exposed to stab other people when discarded after use is urgently needed at present.

### SUMMARY

The present disclosure aims to provide a needle-shielded prefilled syringe. By means of a syringe barrel, a needle, a needle mounting member, a shield unit and a needle guard unit, the technical effect that the needle is encased, and therefore the syringe is prevented from stabbing other people in the discarding process is achieved.

In order to solve the above problem, the present disclosure adopts a technical solution: a needle-shielded prefilled syringe, includes a syringe barrel and a needle, and further includes a needle mounting member, a shield unit and a needle guard unit, wherein
the needle is mounted on the needle mounting member;
the needle protector unit is connected to the needle and part of the needle mounting member in a sleeving manner;
the shield unit is connected to the needle protector unit and part of the syringe barrel in a sleeving manner;
the shield unit includes a shield main body and a front locking element arranged on the shield main body; and
the needle mounting member is arranged at a distal end of the syringe barrel, and a groove is formed between the needle mounting member and the syringe barrel for engaging with the front locking element.

In a further preferred technical solution, the shield unit further includes a rear locking element on the shield main body, wherein
a proximal end of the rear locking element axially jacks up a distal end of a maximum outer diameter position of the needle mounting member.

In a further preferred technical solution, the shield unit further includes wide stopping hooks, wherein
distal ends of the wide stopping hooks are connected with a proximal end of the shield main body, and proximal ends of the wide stopping hooks engage in the groove.

In a further preferred technical solution, the shield unit further includes cantilever members and cantilever openings, wherein
the cantilever openings are formed in the wide stopping hooks; and
the cantilever members are mounted on inner side faces of the cantilever openings.

In a further preferred technical solution, the cantilever openings extend toward distal ends to part of the shield main body.

In a further preferred technical solution, the shield unit further includes narrow stopping hooks, wherein
distal ends of the narrow stopping hooks are connected to the proximal end of the shield main body, and proximal ends of the narrow stopping hooks engage with one side of a proximal end of a maximum outer diameter position of the needle mounting member; and
the narrow stopping hooks are arranged between the two wide stopping hooks.

In a further preferred technical solution, the wide stopping hooks are symmetrically arranged.

In a further preferred technical solution, the narrow stopping hooks are symmetrically arranged.

In a further preferred technical solution, the cantilever members include cantilever elastic members and cantilever locking elements, wherein
distal ends of the cantilever elastic members are arranged on the inner side faces of the cantilever openings;
distal ends of the cantilever locking elements are arranged on proximal ends of the cantilever elastic members; and
when the shield main body is closed, proximal ends of the cantilever locking elements abut against one side of a proximal end of a maximum outer diameter position of the needle mounting member.

In a further preferred technical solution, the cantilever members further include proximal end chamfer structures, wherein
the proximal end chamfer structures are arranged at the proximal ends of the cantilever locking elements, and when the shield main body is opened, the cantilever locking elements pass over the maximum outer diameter position of the needle mounting member.

In a further preferred technical solution, the cantilever members further include distal end chamfer structures, wherein
the distal end chambers are arranged at the distal ends of the cantilever locking elements, and when the shield main body is closed, the cantilever locking elements pass over the maximum outer diameter position of the needle mounting member.

In a further preferred technical solution, the needle mounting member includes a flange member, wherein
when the shield main body is closed, distal ends of the flange member engage with the cantilever locking elements, and proximal ends of the flange member engage with the wide stopping hooks and the narrow stopping hooks.

In a further preferred technical solution, the needle mounting member includes flange chambers, wherein
the flange chambers are arranged at distal ends of a flange member, and when the shield main body is opened, the cantilever locking elements pass over the flange member.

Components of the needle-shielded prefilled syringe further include a sealing stopper and a push rod inserted into the sealing stopper.

A mounting method of the components of the needle-shielded prefilled syringe includes the following steps:
firstly, mounting the needle protector unit into the shield main body;
secondly, mounting the shield main body and the needle protector unit on the needle mounting member;
thirdly, mounting the sealing stopper into the syringe barrel; and
fourthly, mounting the push rod on the sealing stopper.

A medicine filling method of the components of the needle-shielded prefilled syringe includes the following steps:
1. putting the syringe barrel perpendicular to a horizontal plane with an opening facing upward, and pouring quantitative liquid medicines into the syringe barrel;
2. mounting the sealing stopper into the syringe barrel through a cartridge, wherein a maximum outer diameter of the cartridge is less than a minimum inner diameter of the syringe barrel; and
3. mounting the push rod on the sealing stopper.

The present disclosure has the following advantages:
firstly, the needle is encased with the shield unit capable of axially moving, such that the needle cannot be exposed to stab other people, by sliding the shield unit, the needle is exposed in the injection process, while the shield unit cannot disengage from the syringe barrel, the shield unit can be conveniently pushed back directly after injection, and the needle is encased with the shield unit again and then discarded.
Secondly, the plurality of stopping hooks are arranged at the proximal end of the shield unit, the stability of the shield unit after fixation is improved, the stopping hooks cling to a surface of the syringe barrel when the shield unit is opened, and therefore the shield unit can be fixed to the outer circumferential face of the syringe barrel without sliding down in the injection process.
Thirdly, a hollow structure is designed on the stopping hooks, the cantilever members are arranged in the hollow structure to cooperate with the stopping hooks to fix the shield unit, and stress is borne uniformly.
Fourthly, chamfer structure structures corresponding to a main body of the syringe are arranged on the cantilever members, so that the cantilever members can smoothly pass over the flange member without being prone to jamming.

### BRIEF DESCRIPTION OF THE DRAWINGS

FIG. 1 is an exploded diagram of an overall structure of a prefilled syringe according to the present disclosure.
FIG. 2 is a schematic structural diagram illustrating that a shield unit is mounted on a needle mounting member before a needle is encased with a needle protector unit according to the present disclosure.
FIG. 3 is a schematic structural diagram illustrating that a shield unit is mounted on a needle mounting member when a needle is encased with a needle protector unit according to the present disclosure.
FIG. 4 is a schematic structural diagram of a shield unit according to the present disclosure.
FIG. 5 is a cross-sectional view taken along a line AA of FIG. 2.
FIG. 6 is a cross-sectional view taken along a line BB of FIG. 3.
FIG. 7 is an enlarged diagram of a region A of FIG. 5.

In the drawings, the reference signs denote the following components: syringe barrel 11, needle 12, needle mounting member 1, shield unit 2, needle protector unit 3, flange member 101, flange chamfer 102, shield main body 201, wide stopping hook 202, cantilever member 203, cantilever opening 204, narrow stopping hook 205, cantilever elastic member 203a, cantilever locking element 203b, proximal end chamfer structure 203c, and distal end chamfer structure 203d.

### DETAILED DESCRIPTION OF THE EMBODIMENTS

### Defining:

1. Distal end: an end directed toward a needle.
2. Proximal end: an end remote from the needle.
3. Axis: a vertical line passing through a centroid of a regular cross section of an object.
4. Opening direction: a direction along which the needle or a needle protector unit can be exposed in the operating process.
5. Closing direction: a direction along which the needle or the needle protector unit can be encased in the operating process.
6. Sleeving: an act of mounting a first component on an outer surface of a second component when an inner diameter of the first component is larger than an outer diameter of the second component.
7. Arrangement: any means for combining two components together by bonding, engaging, welding, screwing, placing, or the like.
8. Jack-up: an operation that the first component applies force to the second component, such that the second component moves along a direction opposite to the first component.

The following descriptions are only preferred embodiments of the present disclosure, but are not construed as limiting the scope of the present disclosure.

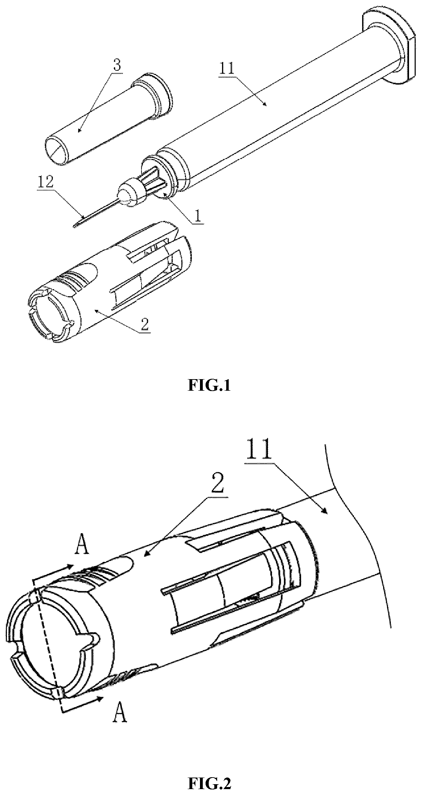

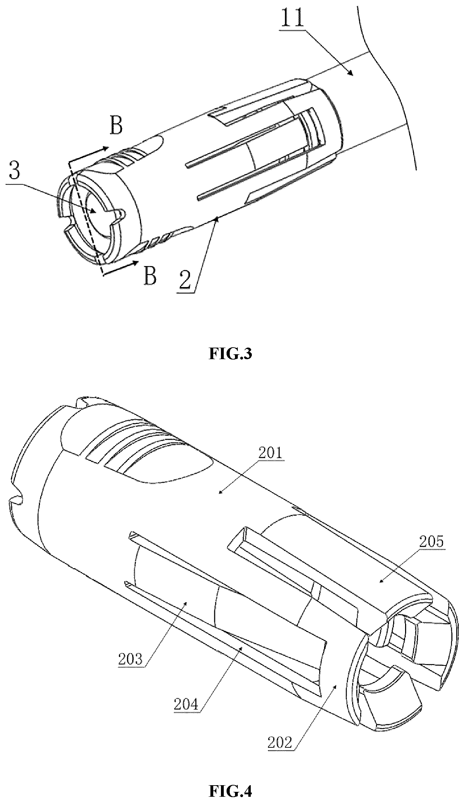

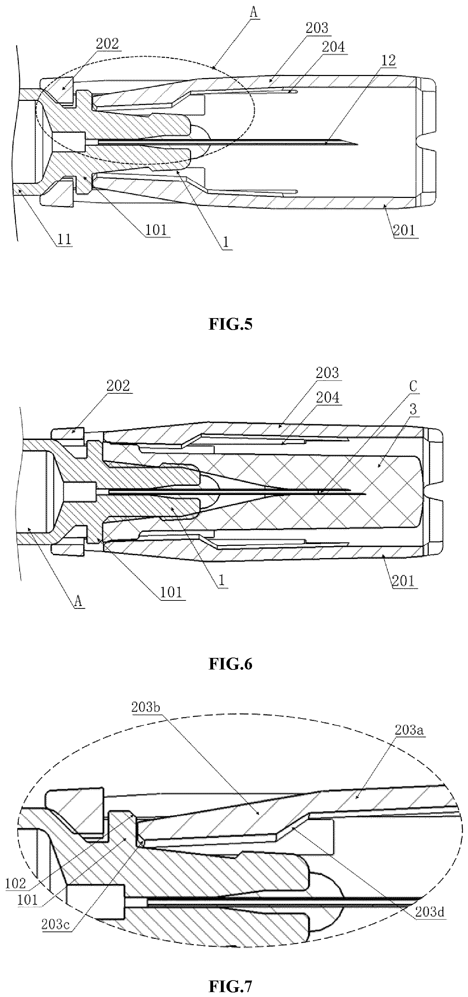

Embodiment: As shown in FIGS. 1-7, a needle-shielded prefilled syringe, includes a syringe barrel 11 and a needle 12, and further includes a needle mounting member 1, a shield unit 2 and a needle protector unit 3, wherein
the needle 12 is mounted on the needle mounting member 1;
the needle protector unit 3 is connected to the needle 12 and part of the needle mounting member 1 in a sleeving manner;
the shield unit 2 is connected to the needle protector unit 3 and part of the syringe barrel 11 in a sleeving manner;
the shield unit 2 includes a shield main body 201 and a front locking element arranged on the shield main body 201; and
the needle mounting member 1 is arranged at a distal end of the syringe barrel 11, and a groove is formed between the needle mounting member and the syringe barrel 11 for engaging with the front locking element.

In this embodiment, a syringe device commonly used in the prior art is formed by the syringe barrel 11 and the needle 12, no push rod is arranged in the syringe barrel 11 in order to conveniently pre-fill medicines, and a downstream manufacturer can voluntarily add an appropriate push rod according to the specification of the syringe barrel 11 after medicine filling, so as to ensure sealing performance; and the shield unit 2 is overall firstly connected to the needle protector unit 3 in a sleeving manner, then the needle 12 is encased with the shield unit and the needle protector unit 3 jointly in the mounting process, and part of the shield unit 2 is connected to the needle mounting member 1 in a sleeving manner.

After medicine filling, a use method of the syringe barrel is as follows:
firstly, the shield unit 2 is pushed to a proximal end, such that the needle protector unit 3 is exposed;
secondly, the needle protector unit 3 is taken down, such that the needle 12 is exposed;
thirdly, the push rod is pushed to perform injection; and
fourthly, the shield unit 2 is pushed to the distal end, and the needle 12 is encased with the shield unit again and then discarded.

In addition, preferably, the shield main body 201 and the front locking element are main components of the shield unit 2, the shield main body 201 is arranged on one side of the distal end of the overall shield unit 2, the front locking element is arranged on one side of the proximal end of the overall shield unit 2, and a minimum inner diameter of the front locking element is less than maximum outer diameters of the needle mounting member 1 and the syringe barrel 11, such that the shield main body 201 may stopping at a position where the needle 12 is encased under the engaging action of the front locking element and the groove in the fourth step, thereby achieving protection.

In addition, the minimum inner diameter of the front locking element may be greater than the maximum outer diameter of the syringe barrel 11 by expanding the front locking element, such that the shield main body 201 slides toward the proximal end in the first step, thereby releasing the encasing effect on the needle 12 before injection.

The shield unit 2 further includes a rear locking element on the shield main body 201, wherein
a proximal end of the rear locking element axially jacks up a distal end of the maximum outer diameter position of the needle mounting member 1.

In this embodiment, the groove is a feature naturally formed by the maximum outer diameter position of the needle mounting member 1 and the syringe barrel 11, when the front locking element engages in the groove, a distal end of the front locking element will axially jack up a proximal end of the maximum outer diameter position of the needle mounting member 1, and meanwhile, the proximal end of the rear locking element axially jacks up the distal end of the maximum outer diameter position of the needle mounting member 1, such that the maximum outer diameter position of the needle mounting member 1 is clamped through the cooperation between the front locking element and the rear locking element, and the shield main body 201 is firmly fixed in the fourth step accordingly without merely relying on acting force generated when the front locking element engages in the groove.

In addition, in the first step, the disengagement of the shield main body from the maximum outer diameter position of the needle mounting member 1 may be done by manually expanding the rear locking element in order to smoothly push the shield main body 201, such that the shield main body 201 is smoothly pushed.

In addition, preferably, in the presence of the needle protector unit 3, the needle protector unit 3 will radially jack up the rear locking element to disengage the rear locking element from the maximum outer diameter position of the needle mounting member 1 accordingly. The advantages of the above arrangement are that when the needle protector unit 3 is in place, the shield main body 201 may be pushed merely through push force without manual expansion, whereas in the fourth step, the needle protector unit 3 has been taken down, as a result, when the rear locking element engages with the maximum outer diameter position of the needle mounting member 1, the position of the shield main body 201 cannot be adjusted merely through the push force, thereby achieving protection on the needle C.

The shield unit 2 further includes wide stopping hooks 202, wherein
distal ends of the wide stopping hooks 202 are connected with the proximal end of the shield main body 201, and proximal ends of the wide stopping hooks 202 engage in the groove.

In this embodiment, the wide stopping hooks 202 are one representation of the front locking element, engaging blocks extending radially are arranged at proximal ends of the wide stopping hooks and used for engaging in the groove, that is, the engaging blocks axially jack up an end face of the proximal end of the maximum outer diameter position of the needle mounting member 1, the wide stopping hooks 202 are elastic, which may expand in inner diameters thereof through bending, such that the wide stopping hooks pass over the groove to release the engaging state.

The shield unit 2 further includes cantilever members 203 and cantilever openings 204, wherein
the cantilever openings 204 are formed in the wide stopping hooks 202; and
the cantilever members 203 are mounted on inner side faces of the cantilever openings 204.

In this embodiment, the cantilever members 203 are one of representations of the rear locking element, the cantilever openings 204 are adjacent to the distal ends of the wide stopping hooks 202, and the cantilever members 203 are arranged on inner side faces of distal ends of the cantilever openings 204 and extend to proximal ends of the cantilever openings, such that a clamping component is formed by the proximal ends of the cantilever openings 204 and the proximal ends of the wide stopping hooks 202 and used for clamping the maximum outer diameter position of the needle mounting member 1 to achieve engaging.

In addition, the cantilever members 203 are mounted by arranging the cantilever openings 204, such that the cantilever members 203 and the wide stopping hooks 202 are integrated; and actually, during preparation, the cantilever members 203 are integrally prepared in the wide stopping hooks 202, such that the cantilever members 203 cannot warp up. The advantages of the above arrangement are that during injection, a nurse will grasp the shield unit 2 at a gesture similar to holding a pencil, use comfort for the nurse is greatly improved due to such corner-free design, and use experience is better compared with that of other products with protruding cantilever members on the market.

The cantilever openings 204 extend toward the distal ends to part of the shield main body 201.

In this embodiment, internal spaces of the cantilever openings are larger after the cantilever openings 204 extend, such that the cantilever members 203 with larger radial lengths may be mounted; and actually, a movement range of radial expansion of the cantilever members 203 is improved, such that the cantilever members can more easily pass over the maximum outer diameter position of the needle mounting member 1 when the shield main body 201 is opened.

The shield unit 2 further includes narrow stopping hooks 205, wherein
distal ends of the narrow stopping hooks 205 are connected with the proximal end of the shield main body 201, and proximal ends of the narrow stopping hooks 205 engage with one side of the proximal end of the maximum outer diameter position of the needle mounting member 1; and
the narrow stopping hooks 205 are arranged between the two wide stopping hooks 202.

In this embodiment, the narrow stopping hooks 205 have the same actual functions as the wide stopping hooks 202, the difference is that no cantilever members 203 are mounted on the narrow stopping hooks 205, the narrow stopping hooks only have the effect of limiting the shield main body 201 from radially moving toward the distal end of the shield main body, however, a spare position between the wide stopping hooks 202 is fully utilized due to such design.

In addition, the two wide stopping hooks 202 are usually arranged at the proximal end of the shield main body 201, balance force may be provided when the narrow stopping hooks 205 are arranged between the wide stopping hooks 202, that is, the shield main body 201 is prevented from shaking when the maximum outer diameter position of the needle mounting member 1 is clamped by the wide stopping hooks 202 and the cantilever members 203.

The wide stopping hooks 202 are symmetrically arranged.

The narrow stopping hooks 205 are symmetrically arranged.

In this embodiment, the wide stopping hooks 202 and the narrow stopping hooks 205 are respectively and symmetrically designed, on one hand, the shield unit 2 is more attractive, and on the other hand, engaging force is kept balanced, such that shaking of the shield main body 201 is relieved.

The cantilever members 203 include cantilever elastic members 203a and cantilever locking elements 203b, wherein
distal ends of the cantilever elastic members 203a are arranged on the inner side faces of the cantilever openings 204;
distal ends of the cantilever locking elements 203b are arranged on proximal ends of the cantilever elastic members 203a; and
when the shield main body 201 is closed, proximal ends of the cantilever locking elements 203b abut against one side of the proximal end of the maximum outer diameter position of the needle mounting member 1.

In this embodiment, each of the cantilever members 203 consists of two parts, the first part is the cantilever elastic member 203a arranged on the cantilever opening 204 and radially bent toward the shield main body 201, and the cantilever locking element 203b is arranged on a tail end face of the cantilever elastic member 203a, such that an end face of the cantilever locking element 203b can abut against a distal end face of the maximum outer diameter position of the needle mounting member 1 and be used for jacking up the distal end face of the maximum outer diameter position of the needle mounting member 1 during closing and applying a friction force on a surface of a syringe main body A during opening, thus achieving a restraining effect.

The cantilever members 203 further include proximal end chamfer structures 203c, wherein
the proximal end chamfer structures 203c are arranged at the proximal ends of the cantilever locking elements 203b, and when the shield main body 201 is opened, the cantilever locking elements 203b pass over the maximum outer diameter position of the needle mounting member 1.

In this embodiment, when the shield main body 201 is axially pushed in the first step, the proximal end chamfer structures 203c axially jack up the distal end face of the maximum outer diameter position of the needle mounting member 1, and radial force will be generated to drive the cantilever elastic members 203a to radially expand to pass over the maximum outer diameter position of the needle mounting member 1, such that the shield main body 201 continues to axially move.

The cantilever members 203 further include distal end chamfer structures 203d, wherein
the distal end chambers 203d are arranged at the distal ends of the cantilever locking elements 203b, and when the shield main body 201 is closed, the cantilever locking elements 203b pass over the maximum outer diameter position of the needle mounting member 1.

In this embodiment, when the shield main body 201 is axially pushed in the fourth step, the distal end chamfer structures 203d axially jack up the proximal end face of the maximum outer diameter position of the needle mounting member 1, and the cantilever elastic members 203a are driven to radially expand to pass over the maximum outer diameter position of the needle mounting member 1, such that the cantilever locking elements 203b engage with the distal end of the maximum outer diameter position of the needle mounting member 1.

The needle mounting member 1 includes a flange member 101, wherein
when the shield main body 201 is closed, distal ends of the flange member 101 engage with the cantilever locking elements 203b, and proximal ends of the flange member 101 engage with the wide stopping hooks 202 and the narrow stopping hooks 205.

In this embodiment, the flange member 101 are one representation of the maximum outer diameter position of the needle mounting member 1, which have the effects that the distal ends thereof are jacked up by the cantilever locking elements 203b and the proximal ends thereof are jacked up by the wide stopping hooks 202 and the narrow stopping hooks 205, and the flange member and the syringe barrel 11 form an annular groove used for engaging with the engaging blocks at the proximal ends of the wide stopping hooks 202 and the narrow stopping hooks 205.

In addition, once being stuck, the flange member 101 cannot disengage from the shield main body no matter how the shield main body 201 rotates.

The needle mounting member 1 includes flange chamfers 102, wherein
the flange chambers 102 are arranged at distal ends of the flange member 101, and when the shield main body 201 is opened, the cantilever locking elements 203b pass over the flange member 101.

In this embodiment, the flange chamfers 102 have the same chamfer structure gradients as the distal end chamfer structures 203d, such that the distal end chamfer structures 203d abut against the flange chamfers 102 in the first step, thereby improving radial expansion force of the cantilever elastic members 203a and conveniently allowing the cantilever members 203 to pass over the flange member 101.

Components of the needle-shielded prefilled syringe further include a sealing stopper and a push rod inserted into the sealing stopper.

In this embodiment, an engaging groove for mounting one end of the push rod is reserved in a rear portion of the sealing stopper.

Amounting method of the components of the needle-shielded prefilled syringe includes the following steps:
firstly, the needle protector unit 3 is mounted into the shield main body 2;
secondly, the shield main body 2 and the needle protector unit 3 are mounted on the needle mounting member 1;
thirdly, the sealing stopper is mounted into the syringe barrel 11; and
fourthly, the push rod is mounted on the sealing stopper.

In this embodiment, the shield main body 2 and the needle protector unit 3 are mounted together in a more straightforward manner, making it convenient for the application in an assembly-line-type mounting process.

A medicine filling method of the components of the needle-shielded prefilled syringe includes the following steps:
1. the syringe barrel 11 is put perpendicular to a horizontal plane with an opening facing upward, and quantitative liquid medicines are poured into the syringe barrel 11;
2. the sealing stopper is mounted into the syringe barrel 11 through a cartridge, wherein a maximum outer diameter of the cartridge is less than a minimum inner diameter of the syringe barrel; and
3. the push rod is mounted on the sealing stopper.

In this embodiment, after the sealing stopper is mounted in the syringe barrel 11, no air is reserved in the syringe barrel 11, and therefore an operator can directly perform injection.

The implementations of the present disclosure are described in detail above with reference to the drawings, however, the present disclosure is not limited to these. Those of ordinary skill in the art can also make various modifications within their knowledge range without departing from the purpose of the present disclosure. These modifications are not inventive, and are protected by patent laws as long as they fall within the scope of the claims of the present disclosure.

## Claims

1. A needle-shielded prefilled syringe, comprising a syringe barrel (11) and a needle (12), and further comprising a needle mounting member (1), a shield unit (2) and a needle protector unit (3), wherein
the needle (12) is mounted on the needle mounting member (1);
the needle protector unit (3) is connected to the needle (12) and part of the needle mounting member (1) in a sleeving manner;
the shield unit (2) is connected to the needle protector unit (3) and part of the syringe barrel (11) in a sleeving manner;
the shield unit (2) comprises a shield main body (201) and a front locking element arranged on the shield main body (201); and
the needle mounting member (1) is arranged at a distal end of the syringe barrel (11), and a groove is formed between the needle mounting member and the syringe barrel (11) for engaging with the front locking element.

2. The needle-shielded prefilled syringe according to claim 1, wherein the shield unit (2) further comprises a rear locking element on the shield main body (201), wherein
a proximal end of the rear locking element axially jacks up a distal end of a maximum external diameter position of the needle mounting member (1).

3. The needle-shielded prefilled syringe according to claim 1, wherein the shield unit (2) further comprises wide stopping hooks (202), wherein
distal ends of the wide stopping hooks (202) are connected with a proximal end of the shield main body (201), and proximal ends of the wide stopping hooks (202) engage in the groove.

4. The needle-shielded prefilled syringe according to claim 3, wherein the shield unit (2) further comprises cantilever members (203) and cantilever openings (204), wherein
the cantilever openings (204) are formed in the wide stopping hooks (202); and
the cantilever members (203) are mounted on inner side faces of the cantilever openings (204).

5. The needle-shielded prefilled syringe according to claim 4, wherein the cantilever openings (204) extend toward distal ends to part of the shield main body (201).

6. The needle-shielded prefilled syringe according to claim 3, wherein the shield unit (2) further comprises narrow stopping hooks (205), wherein
distal ends of the narrow stopping hooks (205) are connected with the proximal end of the shield main body (201), and proximal ends of the narrow stopping hooks (205) engage with one side of a proximal end of a maximum external diameter position of the needle mounting member (1); and
the narrow stopping hooks (205) are arranged between the two wide stopping hooks (202).

7. The needle-shielded prefilled syringe according to claim 3, wherein the wide stopping hooks (202) are symmetrically arranged.

8. The needle-shielded prefilled syringe according to claim 6, wherein the narrow stopping hooks (205) are symmetrically arranged.

9. The needle-shielded prefilled syringe according to claim 4, wherein the cantilever members (203) comprise cantilever elastic members (203a) and cantilever locking elements (203b), wherein
distal ends of the cantilever elastic members (203a) are arranged on the inner side faces of the cantilever openings (204);
distal ends of the cantilever locking elements (203b) are arranged on proximal ends of the cantilever elastic members (203a); and
when the shield main body (201) is closed, proximal ends of the cantilever locking elements (203b) abut against one side of a proximal end of a maximum external diameter position of the needle mounting member (1).

10. The needle-shielded prefilled syringe according to claim 9, wherein the cantilever members (203) further comprise proximal end chamfer structures (203c), wherein
the proximal end chamfer structures (203c) are arranged at the proximal ends of the cantilever locking elements (203b), and when the shield main body (201) is opened, the cantilever locking elements (203b) pass over the maximum external diameter position of the needle mounting member (1).

11. The needle-shielded prefilled syringe according to claim 9, wherein the cantilever members (203) further comprise distal end chamfer structures (203d), wherein
the distal end chambers (203d) are arranged at the distal ends of the cantilever locking elements (203b), and when the shield main body (201) is closed, the cantilever locking elements (203b) pass over the maximum external diameter position of the needle mounting member (1).

12. The needle-shielded prefilled syringe according to claim 9, wherein the needle mounting member (1) comprises a flange member (101), wherein
when the shield main body (201) is closed, distal ends of the flange member (101) engage with the cantilever locking elements (203b), and proximal ends of the flange member (101) engage with the wide stopping hooks (202) and the narrow stopping hooks (205).

13. The needle-shielded prefilled syringe according to claim 9, wherein the needle mounting member (1) comprises flange chambers (102), wherein
the flange chambers (102) are arranged at distal ends of the flange member (101), and when the shield main body (201) is opened, the cantilever locking elements (203b) pass over the flange member (101).
